# FASCICULE DE BREVET EUROPEEN

(11) **EP 1 270 037 B1**
(45) Date de publication et mention de la délivrance du brevet: **29.09.2004**
(21) Numéro de dépôt: 02291521.9
(22) Date de dépôt: 18.06.2002
(51) Int. Cl.: A61M 16/00, F04D 29/44

(54) **Appareil respiratoire avec turbine à pression stabilisée**
Beatmungsvorrichtung beinhaltend eine Turbine mit stabilisiertem Druck
Respiratory apparatus having turbine with stabilized pressure

(30) Priorité: 22.06.2001 FR 0108263
(43) Date de publication de la demande: 02.01.2003
(73) Titulaire: TAEMA, F-92182 Antony Cédex (FR)
(72) Inventeur: Leclerc, Daniel, 75015 Paris (FR)
(74) Mandataire: Pittis, Olivier

(56) Documents cités:
- WO-A-97/10868
- DE-C- 722 059
- GB-A- 1 550 971
- US-A- 3 374 831
- US-A- 4 430 995

## Description

La présente invention se rapporte à un appareil de ventilation artificielle permettant d'alimenter les voies aériennes supérieures d'un patient avec un gaz sous pression, notamment utilisable pour traiter les apnées ou hypopnées du sommeil.

Les troubles respiratoires du sommeil les plus fréquents sont les apnées et les hypopnées.

Habituellement, une apnée est définie comme un trouble du sommeil se caractérisant chez une personne par un arrêt de l'activité respiratoire pendant une période de temps variable durant son sommeil, c'est-à-dire une réduction de plus de 90% de l'amplitude du débit respiratoire normal de cette personne.

Par ailleurs, une hypopnée est définie, quant à elle, comme un trouble du sommeil se caractérisant chez une personne par une diminution de 50% l'amplitude du débit respiratoire de cette personne pendant une période de temps variable durant son sommeil.

Les personnes souffrant de ces problèmes respiratoires durant leur sommeil ont un sommeil perturbé conduisant à une accumulation de fatigue chez celles-ci qui, peut être préjudiciable à une vie professionnelle normale.

On comprend donc qu'il est indispensable de pouvoir traiter ces personnes le plus efficacement possible et ce, sans que le traitement ne soit par lui même trop contraignant ou inconfortable pour ne pas les réveiller ou simplement les empêcher de dormir.

Actuellement, il existe de nombreux types d'appareils de ventilation artificielle permettent d'assurer un traitement ou un diagnostic de ces troubles respiratoires du sommeil ; à ce titre, on peut citer notamment les documents suivants : US-A-5,245,995, US-A-5,148,802, US-A-5,199,424, US-A-4,655,213, US-A-5,335,654, US-A-5,353,788, US-A-5,458,137 et EP-A-505232.

Toutefois, sur les appareils existants se pose le problème des fluctuations ou variations de pression du gaz autour de la valeur de pression de consigne fixée par l'opérateur, à la sortie de la turbine délivrant le gaz sous pression.

Or, ces instabilités de pression sont très nuisibles au patient car, d'une part, elles engendrent des problèmes d'efficacité du traitement médical qui lui est ainsi administré et, d'autre part, provoquent un inconfort notable pour le patient puisque toute variation intempestive de pression du gaz qui lui est délivré, va se répercuter directement sur son tractus respiratoire, en particulier sur ses poumons, et aura aussi pour effet de réveiller le patient ce qui va à l'encontre du but recherché.

En fait, ces instabilité de pression sont dues à des tourbillons ou autres perturbations dans le flux d'air qui rencontrent le becquet de sortie de la turbine.

Une solution à ce problème consistant à tenter de réguler la pression délivrée au moyen de capteurs de débit et de pression a déjà été proposée.

Cependant, cette solution n'est pas vraiment efficace car elle engendre un temps de réaction trop long s'écoulant entre le moment où est réalisé la mesure et le moment où est opéré une action visant à remédier à ces instabilités, en général cette action consistant en une modulation ou une adaptation de la pression délivrée par la turbine, dans un sens ou dans l'autre.

On connaît également le document WO-A-97/10868 qui décrit un appareil de traitement des troubles respiratoires par envoi d'une pression continue de gaz respiratoire, dans lequel une plaque mobile est aménagée en sortie de turbine, ladite plaque servant à réguler le débit de gaz en obturant plus ou moins l'ouverture de passage du gaz.

Par ailleurs les documents GB-A-1 550 971 et DE-A- 722 059 enseignent des turbines avec déflecteurs en sortie destinées à équiper des systèmes de climatisation à air chaud, des turbines à eau ou des collecteurs de poussières à force centrifuge. Toutefois, l'utilisation de telles turbines dans le domaine médical, en particulier pour traiter des patients souffrant de troubles respiratoires, n'y est ni décrite, ni suggérée.

De là, le problème qui se pose est de proposer un appareil amélioré et efficace de traitement ou de diagnostic des troubles respiratoires du sommeil d'un utilisateur, en particulier des apnées ou des hypopnées, ne présentant par de tels problèmes d'instabilité de pression et ne nécessitant pas un temps de réponse aussi long qu'avec les appareils munis de capteurs.

Dit autrement, le but de l'invention est de pouvoir stabiliser, de manière instantanée, la pression du flux de gaz délivré par la turbine d'un appareil médical de manière à ce que les fluctuations de la pression dudit gaz, mesurées en sortie de la turbine, c'est-à-dire à l'entrée du circuit servant à acheminer le gaz jusqu'au patient, encore appelé circuit patient, soient aussi faibles que possible autour d'une valeur de pression souhaitée et choisie par l'opérateur, de préférence les fluctuations de pression autour de ladite valeur de pression de consigne souhaitée ne doivent pas dépasser environ 5 % de ladite valeur, de préférence inférieures à 2.5 %.

En effet, pour permettre d'administrer un traitement efficace à un patient, il est indispensable que la pression délivrée soit aussi stable que possible et qu'elle soit administrée avec le meilleur confort possible.

La solution de l'invention est alors un appareil de traitement ou de diagnostic de trouble respiratoire du sommeil d'un utilisateur comprenant, agencés en série, au moins une turbine pour délivrer un gaz sous pression et au moins un circuit d'acheminement de gaz pour véhiculer ledit gaz sous pression depuis une extrémité amont par laquelle le gaz sous pression entre dans ledit circuit et une extrémité aval par laquelle le gaz sous pression sort dudit circuit, ladite turbine étant munie d'un conduit de sortie de gaz sous pression raccordé à l'extrémité amont du circuit d'acheminement de gaz de manière telle que ladite turbine délivre, lors de son fonctionnement, un flux de gaz respiratoire sous pression dans ledit circuit de gaz, caractérisé en ce qu'au moins un moyen déflecteur de gaz est agencé dans le conduit de sortie de la turbine ou au niveau de l'extrémité amont du circuit d'acheminement de gaz de manière à stabiliser la pression du gaz délivré par la turbine et à obtenir des fluctuations de pression ne dépassant pas 5% d'une valeur de pression de consigne souhaitée.

Selon le cas, l'appareil de l'invention peut comprendre l'une ou plusieurs des caractéristiques techniques suivantes :
- le moyen déflecteur de gaz comporte au moins une cloison agencée coaxialement au conduit de sortie de la turbine.
- la turbine est entraînée par un moteur électrique et de type centrifuge.
- le conduit de sortie de la turbine est de forme tubulaire.
- la cloison formant déflecteur a une épaisseur comprise entre 0.1 et 3 cm, de préférence entre 0.3 et 2 cm.
- il comporte des moyens d'ajustage de niveau de pression de consigne permettant d'ajuster ou de fixer le niveau de pression du gaz délivré par la turbine à une valeur de pression prédéterminée.
- l'extrémité aval du circuit est équipée d'une interface permettant de distribuer le gaz sous pression aux voies aériennes supérieures du patient, en particulier un masque respiratoire nasal ou bucco-nasal.
- il est choisi parmi les BiPAP (à deux niveaux de pression) et les CPAP (à un niveau de pression continue).

Selon un autre aspect, l'invention porte aussi sur un procédé pour stabiliser la pression d'un flux de gaz délivré par la turbine d'un appareil de traitement ou de diagnostic de trouble respiratoire du sommeil d'un utilisateur comprenant, agencés en série, ladite turbine et au moins un circuit d'acheminement de gaz permettant de véhiculer ledit gaz sous pression depuis l'extrémité amont dudit circuit par laquelle le gaz sous pression entre dans ledit circuit et une extrémité aval dudit circuit par laquelle le gaz sous pression ressort dudit circuit, dans lequel :
(a) on choisit ou fixe une valeur de pression de consigne souhaitée,
(b) on fait fonctionner la turbine pour délivrer, par l'intermédiaire de son conduit de sortie de gaz sous pression, un flux de gaz respiratoire à la valeur pression de consigne souhaitée dans l'extrémité amont du circuit d'acheminement de gaz,
(c) on stabilise la pression du gaz délivré à l'étape (b) en utilisant au moins un moyen déflecteur de gaz, agencé dans le conduit de sortie de la turbine ou au niveau de l'extrémité amont du circuit d'acheminement de gaz, de manière à supprimer ou à minimiser toute fluctuation de la pression de gaz par rapport à la valeur de pression de gaz souhaitée choisie à l'étape (a).

Selon le cas, le procédé de l'invention peut comprendre l'une ou plusieurs des caractéristiques suivantes :
- ledit moyen déflecteur de gaz permet de rendre laminaire le flux de gaz délivré par la turbine.
- le gaz respiratoire est de l'air.
- la pression choisie à l'étape (a) est comprise entre 2 et 18 hPa, de préférence entre 8 et 15 hPa.

L'invention va maintenant être mieux comprise grâce à la description détaillée ci-après, faite en références au figures illustratives annexées parmi lesquelles :
- les figure 1a et 1b représentent des schémas possibles d'une turbine d'un appareil selon l'invention,
- la figure 2 montre un graphe des variations ou instabilités de pression enregistrées au cours du temps pour un appareil selon l'art antérieur sans déflecteur en sortie de turbine, et
- la figure 3 montre, quant à elle, un graphe des variations ou instabilités de pression enregistrées au cours du temps pour un appareil selon l'invention, c'est-à-dire un appareil avec déflecteur en sortie de turbine comme schématisé sur les figures 1a (vue de dessus) et 1b (vue de face), et
- la figure 4 schématise un appareil médical selon l'invention.

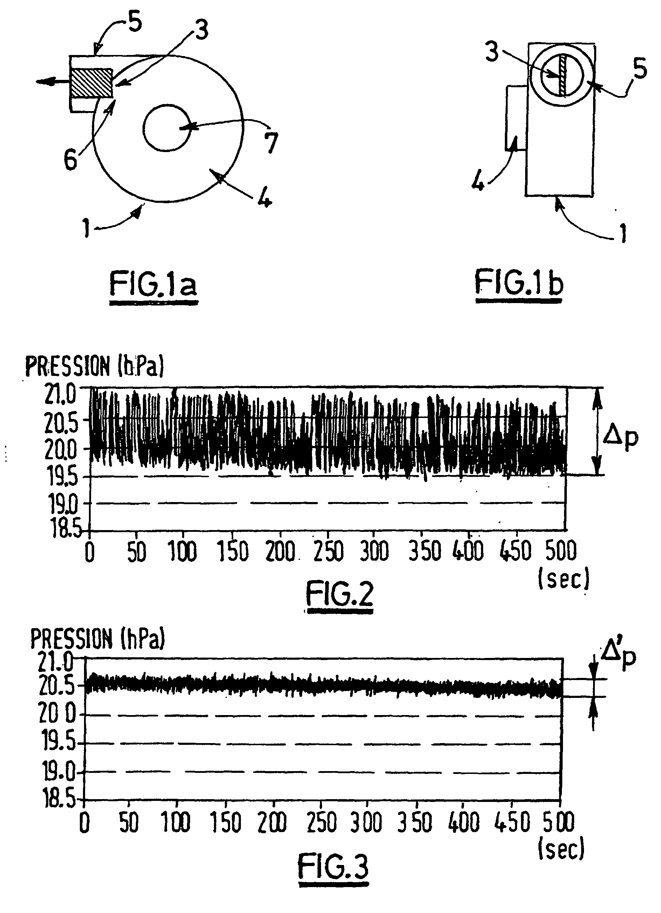

La figure 1a schématise une turbine 1 centrifuge servant à équiper un appareil de traitement ou de diagnostic de trouble respiratoire du sommeil d'un patient, en particulier des apnées et hypopnées du sommeil conforme à la présente invention.

L'agencement de la turbine servant à délivrer un gaz sous pression, tel de l'air, et du circuit d'acheminement de gaz, encore appelé circuit patient, servant à convoyer le gaz sous pression est tout à fait classique, c'est-à-dire que la turbine et ledit circuit d'acheminement de gaz sont agencés en série de manière à ce que la turbine puisse débiter le gaz sous pression à l'extrémité amont du circuit, le gaz étant expulsé hors de la turbine 1 par le conduit 5 de sortie de ladite turbine 1.

Le gaz est ensuite convoyé jusqu'à l'extrémité aval duit circuit par laquelle le gaz sous pression sort dudit circuit en étant administré au patient, via une interface adaptée, tel un masque nasal ou bucco- nasal.

La turbine 1 de type centrifuge comporte, de façon classique, d'un moteur électrique agencé dans la chambre interne 6 d'un carter 4 comportant au moins un orifice d'entrée 7 du gaz (air atmosphérique) dans la turbine 1 et le conduit 5 de sortie du gaz de ladite turbine 1.

Selon l'invention, la turbine 1 comporte un moyen déflecteur 3 de gaz, telle une cloison interne, agencé dans le conduit 5 de sortie de la turbine de manière à séparer en deux le diamètre interne de la turbine 1, comme visible sur la figure 1b et obtenir ainsi un flux laminaire de gaz sous pression directement à la sortie de la turbine 1.

Grâce à cette cloison 3 faisant office de déflecteur, la pression du gaz délivré par la turbine 1 est considérablement stabilisée et ne fluctue pas ou très peu autour de la valeur de pression prescrite ou choisie par l'opérateur.

En d'autres termes, la présence de la cloison 3 interne agencée dans le conduit 5 permet d'éviter la formation de noeuds de pression gazeuse au niveau du conduit sortant de la turbine 1 et donc de l'extrémité amont du circuit d'acheminement de gaz, lesquels noeuds de pression étant à l'origine des instabilités de pression constatées sur les appareils de l'art antérieur.

Préférentiellement, la cloison 3 déborde légèrement dans la chambre 6 interne de la turbine 1.

A titre d'exemple, on peut utiliser une cloison 3 de forme parallélépipédique (rectangulaire) d'épaisseur 0.8 mm et dont la largeur correspond approximativement au diamètre interne du conduit 5.

En outre, dans certains, plusieurs cloisons 3 peut aussi être utilisées.

Des mesures de pression en sortie de turbine ont été réalisées, d'une part, avec un appareil muni d'une turbine sans déflecteur selon l'art antérieur et, d'autre part, avec le même appareil mais muni d'une turbine 1 avec cloison 3 déflecteur selon l'invention et les enregistrements correspondants ayant été obtenus sont donnés en figures 2 et 3, respectivement.

L'appareil utilisé est par exemple un ventilateur du type CPAP commercialisé par la société Taema sous la dénomination Gamme BORA ; la pression de consigne est de 20.5 HPa.

Comme on peut le voir sur l'enregistrement de la figure 2, l'absence de cloison 3 servant de déflecteur en sortie de turbine conduit à une pression fluctuant au cours du temps (en secondes) entre environ 19.5 et 21 HPa.

A l'inverse, l'utilisation d'une cloison 3 servant de déflecteur en sortie de turbine selon l'invention conduit à une pression beaucoup plus stable, ne présentant quasiment plus d'instabilité, puisque celle-ci varie uniquement entre 20.3 et 20.6 HPa. La cloison 3 joue donc pleinement son rôle stabilisateur de pression.

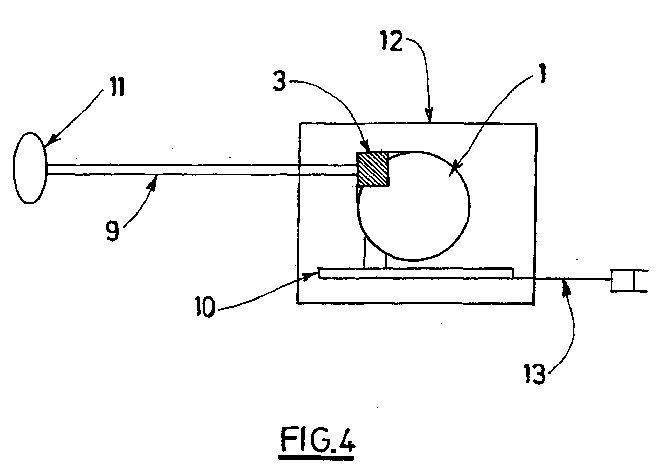

La figure 4 schématise, quant à elle, un appareil selon l'invention comportant une turbine 1 motorisée enfermée dans une enceinte 12 d'insonorisation faisant également office de capotage externe, ladite turbine 1 comportant un déflecteur 3 selon l'invention agencé en amont du circuit 9 d'acheminement de gaz, lequel relie la sortie de la turbine 1 aux voies aériennes supérieures d'un patient, par l'intermédiaire d'un masque respiratoire 11 ou de toute autre interface analogue adaptée.

Le fonctionnement de la turbine 1 est piloté de manière classique par une carte électronique 10 et l'ensemble est alimenté en électricité par des moyens d'alimentation 13 en courant électrique, tel un câble avec prise de branchement secteur.

## Revendications

1. Appareil de traitement ou de diagnostic de trouble respiratoire du sommeil d'un utilisateur comprenant, agencés en série, au moins une turbine (1) pour délivrer un gaz sous pression et au moins un circuit (9) d'acheminement de gaz pour véhiculer ledit gaz sous pression depuis une extrémité amont par laquelle le gaz sous pression entre dans ledit circuit (9) et une extrémité aval par laquelle le gaz sous pression sort dudit circuit (9), ladite turbine (1) étant munie d'un conduit (5) de sortie de gaz sous pression raccordé à l'extrémité amont du circuit d'acheminement de gaz de manière telle que ladite turbine (1) délivre, lors de son fonctionnement, un flux de gaz respiratoire sous pression dans ledit circuit de gaz, **caractérisé en ce qu'**au moins un moyen déflecteur (3) de gaz est agencé dans le conduit (5) de sortie de la turbine ou au niveau de l'extrémité amont du circuit d'acheminement (9) de gaz de manière à stabiliser la pression du gaz délivré par la turbine (1) et à obtenir des fluctuations de pression ne dépassant pas 5% d'une valeur de pression de consigne souhaitée.

2. Appareil selon la revendication 1, **caractérisé en ce que** le moyen déflecteur (3) de gaz comporte au moins une cloison (3) agencée coaxialement au conduit (5) de sortie de la turbine (1) en divisant en deux le diamètre interne dudit conduit (5).

3. Appareil selon l'une des revendications 1 ou 2, **caractérisé en ce que** la turbine (1) est entraînée par un moteur électrique et de type centrifuge.

4. Appareil selon l'une des revendications 1 à 3, **caractérisé en ce que** le conduit (5) de sortie de la turbine (1) est de forme tubulaire et la cloison (3) est agencée diamétralement dans le conduit (5).

5. Appareil selon l'une des revendications 1 à 4, **caractérisé en ce que** la cloison (3) formant déflecteur a une épaisseur comprise entre 0.1 et 3 cm, de préférence entre 0.3 et 2 cm.

6. Appareil selon l'une des revendications 1 à 5, **caractérisé en ce qu'**il comporte des moyens d'ajustage de niveau de pression permettant d'ajuster ou de fixer le niveau de pression du gaz délivré par la turbine (1) à une valeur de pression de consigne prédéterminée.

7. Appareil selon l'une des revendications 1 à 6, **caractérisé en ce que** l'extrémité aval du circuit est équipée d'une interface permettant de distribuer le gaz sous pression aux voies aériennes supérieures du patient, en particulier un masque respiratoire nasal ou bucco-nasal.

8. Appareil selon l'une des revendications 1 à 6, **caractérisé en ce qu'**il est choisi parmi les BPAP et les CPAP.

## Patentansprüche

1. Vorrichtung zur Behandlung oder zur Diagnose von Atemstörungen eines Verwenders während des Schlafs, die Folgendes, hintereinander angeordnet, umfasst: mindestens eine Turbine (1) zur Lieferung eines Druckgases und mindestens einen Kreislauf (9) zur Zuführung von Gas zur Beförderung des Druckgases zwischen einem stromaufwärtigen Ende, durch das das Druckgas in den Kreislauf (9) eintritt, und einem stromabwärtigen Ende, durch das das Druckgas den Kreislauf (9) verlässt, wobei die Turbine (1) mit einer Druckgasausgangsleitung (5) versehen ist, die so an dem stromaufwärtigen Ende des Gaszuführkreislaufs angeschlossen ist, dass die Turbine (1) bei Betrieb einen druckbeaufschlagten Beatmungsgasstrom in den Gaskreislauf einleitet, **dadurch gekennzeichnet, dass** mindestens ein Gasablenkmittel (3) in der Ausgangsleitung (5) der Turbine oder am stromaufwärtigen Ende des Gaszuführkreislaufs (9) angeordnet ist, um den Druck des durch die Turbine (1) zugeführten Gases zu stabilisieren und Druckschwankungen zu erhalten, die nicht 5% eines gewünschten Drucksollwerts überschreiten.

2. Vorrichtung nach Anspruch 1, **dadurch gekennzeichnet, dass** das Gasablenkmittel (3) mindestens eine Trennwand (3) umfasst, die koaxial zur Ausgangsleitung (5) der Turbine (1) angeordnet ist und dabei den Innendurchmesser der Leitung (5) in zwei teilt.

3. Vorrichtung nach Anspruch 1 oder 2, **dadurch gekennzeichnet, dass** die Turbine (1) durch einen elektrischen Zentrifugalmotor angetrieben wird.

4. Vorrichtung nach einem der Ansprüche 1 bis 3, **dadurch gekennzeichnet, dass** die Ausgangsleitung (5) der Turbine (1) röhrenförmig ist und die Trennwand (3) diametral in der Leitung (5) angeordnet ist.

5. Vorrichtung nach einem der Ansprüche 1 bis 4, **dadurch gekennzeichnet, dass** die eine Ablenkvorrichtung bildende Trennwand (3) eine Dicke zwischen 0,1 und 3 cm, vorzugsweise zwischen 0,3 und 2 cm, aufweist.

6. Vorrichtung nach einem der Ansprüche 1 bis 5, **dadurch gekennzeichnet, dass** sie Mittel zur Einstellung der Druckhöhe aufweist, wodurch die Druckhöhe des durch die Turbine (1) gelieferten Gases auf einen vorbestimmten Drucksollwert eingestellt werden kann.

7. Vorrichtung nach einem der Ansprüche 1 bis 6, **dadurch gekennzeichnet, dass** das stromabwärtige Ende des Kreislaufs eine Grenzfläche aufweist, die es gestattet, das Druckgas auf die oberen Luftwege des Patienten zu verteilen, insbesondere eine Atemnasen- oder Atmenhalbmaske.

8. Vorrichtung nach einem der Ansprüche 1 bis 6, **dadurch gekennzeichnet, dass** sie unter den BIPAP und den CPAP ausgewählt ist.

## Claims

1. Apparatus for treatment or diagnosis of breathing problems occurring during a user's sleep, said apparatus comprising, arranged in series, at least one turbine (1) for delivering a compressed gas and at least one gas circuit (9) for conveying said compressed gas from an upstream end, through which the compressed gas enters said circuit (9), to a downstream end, through which the compressed gas leaves said circuit (9), said turbine (1) being provided with a compressed gas outlet conduit (5) connected to the upstream end of the gas circuit in such a way that said turbine (1), when it is in operation, delivers a flow of compressed respiratory gas into said gas circuit, **characterized in that** at least one gas deflector means (3) is arranged in the outlet conduit (5) of the turbine or at the upstream end of the gas circuit (9) in such a way as to stabilize the pressure of the gas delivered by the turbine (1) and to obtain pressure fluctuations not exceeding 5% of a desired setpoint pressure value.

2. Apparatus according to Claim 1, **characterized in that** the gas deflector means (3) comprises at least one partition (3) arranged coaxially with respect to the outlet conduit (5) of the turbine (1) and dividing in two the internal diameter of said conduit (5).

3. Apparatus according to either of Claims 1 and 2, **characterized in that** the turbine (1) is driven by an electric motor and of the centrifuge type.

4. Apparatus according to one of Claims 1 to 3, **characterized in that** the outlet conduit (5) of the turbine (1) is of tubular shape, and the partition (3) is arranged diametrically in the conduit (5).

5. Apparatus according to one of Claims 1 to 4, **characterized in that** the partition (3) forming the deflector has a thickness of between 0.1 and 3 cm, preferably of between 0.3 and 2 cm.

6. Apparatus according to one of Claims 1 to 5, **characterized in that** it comprises pressure level adjustment means making it possible to adjust or fix the pressure level of the gas delivered by the turbine (1) to or at a predetermined setpoint pressure value.

7. Apparatus according to one of Claims 1 to 6, **characterized in that** the downstream end of the circuit is equipped with an interface allowing the compressed gas to be distributed to the upper airways of the patient, in particular a nasal or oronasal breathing mask.

8. Apparatus according to one of Claims 1 to 6, **characterized in that** it is chosen from BPAP and CPAP apparatus.
